# EUROPEAN PATENT SPECIFICATION

(11) **EP 1 498 119 B1**
(45) Date of publication and mention of the grant of the patent: **15.11.2006**
(21) Application number: 04252434.8
(22) Date of filing: 27.04.2004
(51) Int. Cl.: A61K 31/201, A61P 11/02, A23L 1/30

(54) **Use of conjugated linoleic acid for treating colds**
Verwendung von konjugierter Linolsäure zur Behandlung der Erkältung
Utilisation d'acides linoléiques conjugués pour traiter le rhume

(30) Priority: 16.07.2003 US 619549
(43) Date of publication of application: 19.01.2005
(73) Proprietor: LODERS CROKLAAN B.V., 1521 AZ Wormerveer (NL)
(72) Inventor: O'Shea, Marianne, Channahon, Illinois 60410-5249 (US); Schmid, Ulrike, 1521 AZ Wormerveer (NL); Mohede, Ingrid Celestina Maria, 1521 AZ Wormerveer (NL); Cain, Frederick William, 1521 AZ Wormerveer (NL)
(74) Representative: Stevens, Ian Edward

(56) References cited:
- EP-A- 0 396 251
- EP-A- 1 097 708
- WO-A1-03/090739
- US-A- 5 585 400
- US-A- 6 077 868
- ANTHONY M: "Individual free fatty acids and migraine." CLINICAL AND EXPERIMENTAL NEUROLOGY. 1978, vol. 15, 1978, pages 190-196, XP008038724 ISSN: 0196-6383
- KELLEY D S ET AL: "DIETARY CONJUGATED LINOLEIC ACID DID NOT ALTER IMMUNE STATUS IN YOUNG HEALTHY WOMEN" LIPIDS, CHAMPAIGN, IL, US, vol. 35, no. 10, October 2000 (2000-10), pages 1065-1071, XP001118595 ISSN: 0024-4201

## Description

This invention relates to a method of preventing or treating a common cold. In particular, the invention relates to the use of certain compounds and their derivatives for this purpose.

The term "common cold" is a well-known term used by both medical practitioners and the public in general and refers to illnesses caused by a viral infection which is located in the nose, but which may also involve the sinuses, ears and bronchial tubes. The symptoms of the common cold include sneezing, a runny nose, nasal obstruction or stuffiness, sore or itchy throat, cough, hoarseness and mild general symptoms such as headache, fever, chilliness and a general feeling of being unwell. It is known that the common cold is not a single entity, but rather is a group of diseases caused by members of several families of viruses. More than 200 viruses are known to cause the symptoms of the common cold. The most important viruses are the coronaviruses, picomaviruses, rhinoviruses, coxackieviruses and adenoviruses. Other viruses associated with the common cold include parainfluenza viruses, respiratory syncytial viruses and enteroviruses.

Numerous methods of treating the common cold have been described. These include, for example, WO 02/09699 which describes a method of treating a common cold comprising administration of a flavonoid alone or in combination with a metal, and WO 02/40023 which discloses the use of an NK3 antagonist in the treatment of the common cold.

Although many remedies exist for the common cold and its symptoms, there remains a need for treatments of the common cold, and the symptoms of the common cold, that are relatively inexpensive, yet safe and effective.

US 4,851,437 asserts that various tung oil compositions are useful for treating body deficiencies as varied as cancer, AIDS, ageing and schizophrenia, but fails to support these assertions with any data.

WO 03/090739 describes the use of conjugated linoleic acid or derivatives thereof to prevent or cure influenza, to boost the effects of an influenza vaccination and/or to alleviate the effects of an influenza vaccination in humans and/or animals.

EP 0 396 251 describes the use of arachidonic acid, or an analogue thereof capable of interfering with the binding of the cytokine to the cellular receptor, for the cytokine, in the preparation of a medicament for use in the treatment of a disease state associated with the endogenous presence and/or production of a cytokine. It is stated that arachidonic acid or an analogue thereof can be used to alleviate the symptoms of the common cold or of influenza.

EP 1 097 708 describes the use of trans- trans isomers of conjugated linoleic acid to provide anti-inflammatory properties in foods or food supplements.

US 5,674,901 and US 5,827,885 disclose the use of conjugated linoleic acid (CLA) to maintain or elevate CD-4 and CD-8 cell levels to prevent or alleviate the adverse effects of TNF or a virus. Fifteen broad families of viruses are listed covering viruses as diverse as HIV and hepatitis B, but the only example given is the treatment of fowl pox virus. Neither rhinoviruses nor the treatment of the common cold is mentioned in either document.

According to the present invention in a first aspect, there is provided the use of conjugated linoleic acid or a derivative thereof as defined in claim 1 in the manufacture of a composition for the treatment or amelioration of symptoms of the common cold in a mammal.

In another aspect, the invention provides the use of a conjugated fatty acid or a derivative thereof according to the claims in the manufacture of a composition for the prevention or treatment of the common cold, or the treatment or amelioration of symptoms of the common cold in a mammal, wherein the conjugated fatty acid or derivative thereof is conjugated linoleic acid or as derivative thereof, according to the claims the conjugated linoleic acid or derivative thereof comprises trans10, cis12 and cis9, trans11 isomers and the weight ratio of trans 10, cis 12 isomer to cis 9, trans 11 isomer is at least 1.2:1

The present invention further involves the use of conjugated linoleic acid or a derivative thereof as defined in claim 1 in the manufacture of a composition for the prevention or treatment of the common cold, or the treatment or amelioration of symptoms of the common cold in a mammal. The invention also contemplates conjugated linoleic acid or a derivative thereof as defined in claim 1 for use in the prevention or treatment of the common cold, or the treatment or amelioration of symptoms of the common cold in a mammal.

The term "preventing or treating a common cold in a mammal, or of treating or ameliorating the symptoms of a common cold in a mammal", includes prophylaxis and full or partial treatment. It may also include reducing the symptoms of the common cold, ameliorating the symptoms of the common cold, reducing the severity of the common cold or its symptoms, reducing the incidence of the common cold, or any other change in the condition of the patient, which improves the therapeutic outcome. In a preferred aspect of the invention, the use of the invention has at least the effect of reducing the recovery time after a common cold.

Symptoms of the common cold include one or more of sneezing, a runny nose/rhinitis, nasal obstruction (blocked nose or stuffiness), sore or itchy throat, coughing, hoarseness, asthma exacerbation and mild general symptoms such as headache, fever, chilliness and a general feeling of being unwell (or malaise). The invention may therefore comprise the treatment or prevention of one or more of sneezing, runny nose, nasal obstruction, sore throat, itchy throat, coughing, hoarseness, asthma exacerbation, headache, fever, chilliness and malaise, especially sore throat.

The invention is particularly useful when the common cold is caused by a coronavirus or a rhinovirus. In particular, the present invention is directed to the treatment, including prophylaxis, of a viral infection in a human, which is caused by the human rhinovirus (HRV), or a coronavirus. The viruses include the various different serogroups.

Without wishing to be bound by theory, it is believed that the conjugated fatty acid may exert activity, at least in part, against viruses responsible for the common cold by influencing ICAM-1 (intercellular adhesion molecule-1, which is a receptor for certain viruses), and/or by affecting CD58 cell levels.

Mammals for treatment according to the invention are not limited and include, for example, cats, dogs, cattle, sheep and horses. Preferably, the mammal is a human.

In the invention, the mammal is typically administered a composition comprising the conjugated linoleic acid or a derivative thereof as defined in claim 1. The conjugated linoleic acid may be used in the form of the free acid. Derivatives of conjugated linoleic acid are salts and esters thereof, or a mixture of two or more of these materials. Salts are non-toxic, pharmaceutically acceptable and/or acceptable for use in food products and/or pharmaceuticals and include, for example, salts with alkali metals and alkaline earth metals such as sodium, calcium and magnesium, preferably sodium. Esters are mono-, di- and tri- glycerides and mixtures thereof, and C₁ to C₆ alkyl esters (where the alkyl group can be straight chain or branched).

Conjugated linoleic acid and derivatives thereof according to claim 1 may be used in the invention alone (i.e., as a single conjugated linoleic acid) or as mixtures of two or more derivatives of conjugated linoleic acid. Suitable mixtures also include mixtures of conjugated linoleic acid with one or more derivatives of the same fatty acid.

Conjugated linoleic acid (CLA), which is the conjugated fatty acid for use in the present invention, comprises a mixture of two or more different isomers including cis, trans; trans, cis. The isomers comprise the trans10, cis12 and cis9, trans 11 isomers, including these isomers in relatively pure form, as well as mixtures with each other and/or mixtures with other isomers. More preferably, the conjugated linoleic acid or derivative thereof comprises trans10, cis12 and cis9, trans11 isomers and the weight ratio of trans10, cis12 isomer to cis9, trans11 isomer is at least 1.2:1, such as 1.3:1, even more preferably at least 1.5:1, e.g., in the range 1.5:1 to 100:1 or 1.5:1 to 10:1, such as a 60:40 or 80:20 mixture of the trans10, cis12: cis9, trans 11 isomers. Particularly preferred are compositions comprising the trans 10, cis 12 isomer as the major isomer component i.e., present in an amount of at least 55 %, preferably at least 60 %, more preferably at least 70 %, even more preferably at least 75 %, most preferably at least 80 %, such as at least 90 % or even 100 % by weight based on the total amount of conjugated linoleic acid.

Conjugated fatty acids can be produced in conventional ways. For example, conjugated linoleic acid can be produced by known methods, such as that described in EP-A-902082. Conjugated linoleic acid products that are enriched in one or more isomers are disclosed in WO 97/18320.

The conjugated linoleic acid is preferably used and/or administered in the form of a composition. Suitable compositions are, preferably, a pharmaceutical composition, a foodstuff or a food supplement. These compositions provide a convenient form in which to deliver the conjugated fatty acid. Compositions of the invention may comprise an antioxidant in an amount effective to increase the stability of the conjugated linoleic acid or derivative thereof with respect to oxidation.

The amount of conjugated linoleic acid or derivative thereof that is administered in the method of the invention or that is for administration in the use of the invention is preferably from 0.1g to 20g (more preferably 0.1g to 10g, such as 0.5g to 5g) of conjugated linoleic acid or derivative thereof per day. Suitable compositions can be formulated accordingly.

A preferred composition according to the invention is a foodstuff. Food products (which term includes animal feed) contain a fat phase, wherein the fat phase contains the product of the invention. The foodstuffs are optionally used as a blend with a complementary fat. For example, the blend may comprise 0.3 - 95 wt %, preferably 2-80 wt %, most preferably 5-40 wt % of the product of the invention and 99.7 - 5 wt %, preferably 98-20 wt %, most preferably 95-60 wt % of a complementary fat selected from: cocoa butter, cocoa butter equivalents, palm oil or fractions thereof, palmkernel oil or fractions thereof, interesterified mixtures of said fats or fractions thereof, or liquid oils, selected from: sunflower oil, high oleic sunflower oil, soybean oil, rapeseed oil, cottonseed oil, fish oil, safflower oil, high oleic safflower oil, maize oil and MCT-oils. Examples of suitable foodstuffs include those selected from the group consisting of margarines, fat continuous or water continuous or bicontinuous spreads, fat reduced spreads, confectionery products such as chocolate or chocolate coatings or chocolate fillings or bakery fillings, ice creams, ice cream coatings, ice cream inclusions, dressings, mayonnaises, cheeses, cream alternatives, dry soups, drinks, cereal bars, sauces, snack bars, dairy products, clinical nutrition products and infant formulations.

Other examples of compositions are pharmaceutical compositions, such as in the form of tablets, pills, capsules, caplets, multiparticulates including: granules, beads, pellets and micro-encapsulated particles; powders, elixirs, syrups, suspensions and solutions. Pharmaceutical compositions will comprise a pharmaceutically acceptable diluent or carrier. Pharmaceutical compositions are preferably adapted for administration parenterally (e.g., orally). Orally administrable compositions may optionally comprise one or more of a decongestant, an anti-histamine and an anti-pyretic (e.g., paracetamol). Orally administrable compositions may be in solid or liquid form and may take the form of tablets, powders, suspensions and syrups. Optionally, the compositions comprise one or more flavouring and/or colouring agents. Pharmaceutical compositions may be formulated in other ways, such as for administration to a mucosa, including a nasal mucosa; such compositions may optionally comprise at least one humectant. Humectants are capable of absorbing or retaining water and include, for example, mineral oils, vegetable oils, soothing agents, cellulose derivatives, sugars, alcohols, polymers, or membrane conditioners, in particular glycerol, sorbitol, propylene glycol, glycerine, and polyethylene glycols.

Pharmaceutically acceptable carriers suitable for use in such compositions are well known in the art of pharmacy. The compositions of the invention may contain 0.1-99% by weight of conjugated linoleic acid. The compositions of the invention are generally prepared in unit dosage form. Preferably the unit dosage of conjugated linoleic acid is from 1mg to 1000mg (more preferably from 100mg to 750mg). The excipients used in the preparation of these compositions are the excipients known in the art.

Further examples of product forms for the composition are food supplements, such as in the form of a soft gel or a hard capsule comprising an encapsulating material selected from the group consisting of gelatin, starch, modified starch, starch derivatives such as glucose, sucrose, lactose and fructose. The encapsulating material may optionally contain crosslinking or polymerizing agents, stabilizers, antioxidants, light absorbing agents for protecting light-sensitive fills, preservatives and the like. Preferably, the unit dosage of conjugated linoleic acid in the food supplements is from 1mg to 1000mg (more preferably from 100mg to 750mg).

The following examples illustrate the invention. In the examples and throughout this specification, all percentages, parts and ratios are by weight unless indicated otherwise.

### Examples

### Brief description of the Drawings

Figure 1 is a plot of Jackson Score against number of days for the patients treated with the placebo (upper line, black) and those treated with CLA (lower line, grey).
Figure 2 shows the results of tests carried out in the afternoon and shows Total Symptom Score against number of days. Results for the group of people treated with CLA are shown on the left in grey (Series 1) and for people given the placebo are shown on the right in dark grey (Series 2).
Figure 3 corresponds to Figure 2 but relates to tests carried out in the morning.
Figure 4 is a graph showing total symptom score and individual symptoms for the CLA group (light grey bars) and the placebo group (dark grey bars).

### Examples 1 and 2

### In vivo Study

### Protocol

45 human volunteers suffering from the common cold were used in the study. 21 subjects were administered conjugated linoleic acid (CLA) and the other 24 subjects were given a placebo.

The 21 subjects underwent pre-treatment with CLA at a level of 1.7 g/day for 4 weeks. 24 subjects underwent pre-treatment with placebo (HOSF; high oleic sunflower acids).

All subjects were inoculated at day 0 by intranasal exposure to human Rhinovirus (HRV) and the two groups were monitored daily for the next 5 days. The effects of the two types of treatment were determined by using the Jackson Score (validated in severity of symptoms) and the effects of the symptoms on its own on day 0 - 5.

Symptoms were assessed using a Jackson Score validated in severity from 0=absent to 3=very severe. The following symptoms were rated:

| | |
|---|---|
| Runny nose | Stuffiness |
| Sneezing | Sore throat |
| Cough | Headache |
| Malaise | Chilliness |

### Example 1

### Recovery After The Common Cold

The primary endpoint for the study was the frequency of clinical colds defined in accordance with the modified Jackson criteria. A subject will be considered to have a clinical cold if he/she has a cumulative symptom score of 6 or greater over the five days post-challenge (adjusted for any baseline symptom) and either reports runny nose on 3 post challenge days or responds "yes" to the question on day 5 post challenge whether he/she feels that he/she has had a cold during the previous 5 days.

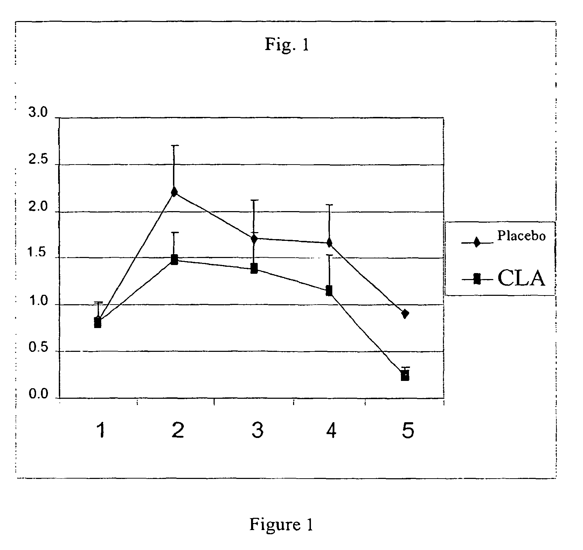

Figure 1 is a plot of Jackson Score against number of days for the patients treated with the placebo (upper line, black) and those treated with CLA (lower line, grey).

The results show that at day 2 already, the severity of the common cold is lower in the CLA treated people. Given the fact that the difference already occurred at day 2 demonstrated the positive effect of CLA.

### Example 2

### Treatment of Symptoms

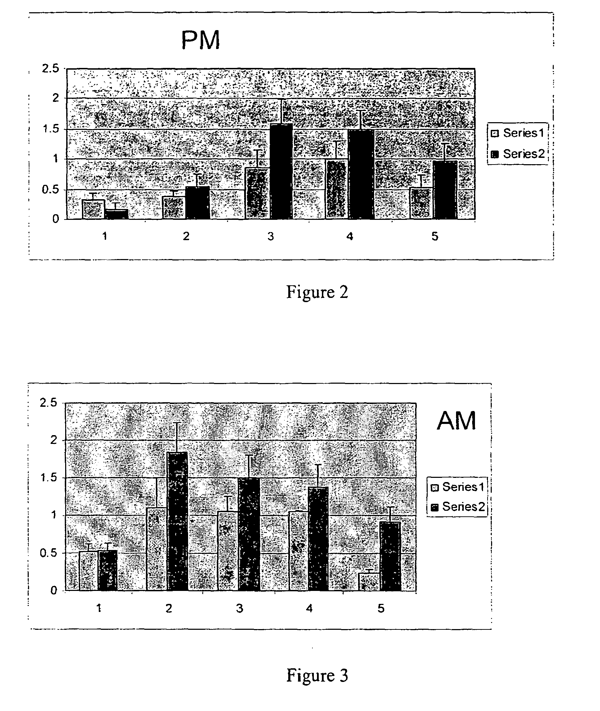

Total symptoms of the individuals were checked in the morning (am) and in the afternoon (pm). The results of the test in the afternoon are shown in Figure 2 and the results of the test in the morning are shown in Figure 3; both plots are of Total Symptom Score against number of days. In Figures 2 and 3, results for the group of people treated with CLA are shown on the left in grey (Series 1) and for people given the placebo are shown on the right in dark grey (Series 2).

The results showed that the total symptom score at day 2 until the end of the study is lower in the CLA treated people.

### Example 3

### Assessment of total symptoms separately

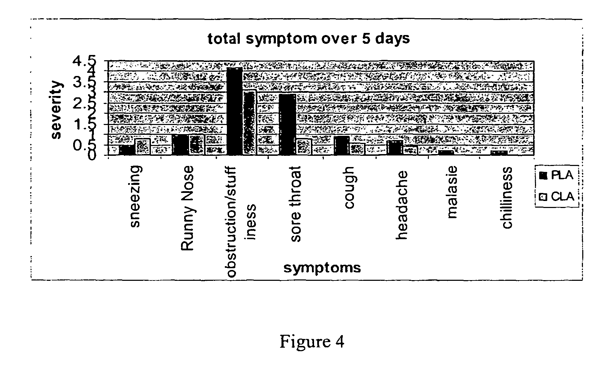

The symptoms assessed were sneezing, runny nose, obstruction, stuffiness, sore throat, cough, headache, malaise and chilliness. The total symptom score was determined by adding the scores for the symptoms during the 5 day period. Figure 4 is a graph showing total symptom score and individual symptoms for the CLA group (light grey bars) and the placebo group (dark grey bars).

The results in Figure 4 show that the severity of the symptoms assessed separately is very clearly lower in the CLA group (CLA) than in the placebo group (PLA).

### Examples 4 to 10

### In vivo study II

### Protocol

The following protocol was used in all of Examples 4 to 10.

76 human volunteers suffering from the common cold were used in the study. 43 subjects were administered conjugated linoleic acid (CLA) and the other 33 subjects were given a placebo. The CLA used contained a 40:60 mixture of cis9, trans 11: trans 10, cis 12 isomers.

The 43 subjects underwent pre-treatment with CLA at a level of 2 g/day for 4 weeks. 33 subjects underwent pre-treatment with placebo (HOSF; high oleic sunflower acids).

All subjects were inoculated at day 0 by intranasal exposure to human Rhinovirus (HRV) and the two groups were monitored daily for the next 5 days. The effects of the two types of treatment were determined by using the Jackson Score (validated in severity of symptoms) and the effects of the symptoms on its own on day 0-5.

Symptoms were assessed using a Jackson Score validated in severity from 0=absent to 3=very severe. The following symptoms were rated:

| | |
|---|---|
| Runny nose | Stuffiness |
| Sneezing | Sore throat |
| Cough | Headache |
| Malaise | Chilliness |

### Example 4

### Infection

Seven out of the 43 volunteers were not infected in the group treated with CLA. All of the volunteers were infected in the group treated with the placebo.

### Example 5

### Total Jackson score over 5 days

The Jackson score as described above was monitored on two time points AM and PM for each of the five days post inoculation. The total score for each individual was compiled and then the average between the individuals in the treatment group compared to placebo was calculated. The average total score for the group treated with CLA was 8.72, and the average total score for the group treated with the placebo was 9.30. There is a reduction of about 7% in total Jackson score over 5 days in the CLA group.

### Example 6

### Jackson cold severity

The Jackson cold has a severity marking from mild to severe and was monitored on two time points AM and PM for each of the five days post inoculation. The total score (from 10) for each individual was compiled and then the average between the individuals in the treatment group compared to the placebo was calculated. The average score was 1.09 for the placebo group and 0.975 for the CLA group. There is a reduction of about 12% in the CLA group.

### Example 7

### Total symptom scores over 5 days

The symptoms assessed were sneezing, runny nose, obstruction, stuffiness, sore throat, cough, headache, malaise and chilliness. The total symptom score was determined by adding the scores for the symptoms during the 5 day period. The results were 12.1 for the placebo group and 11.3 for the CLA group. The results show that the severity of the symptoms assessed separately is lower in the CLA group than in the placebo group (PLA).

### Example 8

### Total sore throat for 5 days

The symptom sore throat was recorded in three rankings (1 to 3 for mild to severe) and was monitored on two time points AM and PM for each of the five days post inoculation. The total score for each individual was compiled and then the average between the individuals in the treatment group compared to the placebo group was calculated. The results were 3.1 for the placebo group and 2.5 for the CLA group.

### Example 9

### Total sneezing in 5 days

The symptom sneezing was recorded in three rankings (1 to 3 for mild to severe) and was monitored on two time points AM and PM for each of the five days post inoculation. The total score for each individual was compiled and then the average between the individuals in the treatment group compared to placebo was calculated. The results were 1.2 for the placebo group and 0.5 for the CLA group.

### Example 10

### Total runny nose in 5 days

The symptom runny nose was recorded in three rankings (1-3 mild- severe) and was monitored on two time points AM and PM for each of the five days post inoculation. The total score for each individual was compiled and then the average between the individuals in the treatment group compared to the placebo group was calculated. The results were 2.14 for the placebo and 2.025 for CLA. The results show that the symptom of runny nose assessed over 5 days is very clearly lower in the CLA group than in the placebo group.

### Example 11

The following is an example of a filled gelatin capsule according to the invention. A composition comprising 60 % by weight trans10, cis12 conjugated linoleic acid and 40 % by weight cis9, trans 11 linoleic acid is encapsulated into a gelatin capsule according to methods well-known in the art. The resulting encapsulated product contains 500 mg of the mixture of conjugated linoleic acid isomers and one tablet can be taken up to four times daily by an adult human.

## Claims

1. The use of conjugated linoleic acid or a derivative thereof selected from salts, mono-,di-, tri-glycerides and C₁-C₆ alkyl esters thereof in the manufacture of a composition for the treatment or amelioration of symptoms of the common cold in a mammal, wherein the conjugated linoleic acid or derivative thereof comprises a mixture of trans 10, cis 12 and cis 9, trans 11 isomers.

2. Use as claimed in Claim 1, wherein the symptoms are selected from one or more of sneezing, runny nose, nasal obstruction, sore throat, itchy throat, coughing, hoarseness, asthma exacerbation, headache, fever, chilliness, malaise and sore throat.

3. Use as claimed in Claim 2, wherein the symptom is sore throat.

4. Use as claimed in any one of Claims 1 to 3, wherein the mammal is a human.

5. Use as claimed in any one of Claims 1 to 4, wherein the composition is a pharmaceutical composition, a foodstuff or a food supplement.

6. Use as claimed in any one of Claims 1 to 5, wherein the common cold is caused by a coronavirus or a rhinovirus.

7. Use as claimed in any one of Claims 1 to 6, wherein the composition comprises an amount of conjugated linoleic acid or derivative thereof such that the mammal receives a dose of 0.1 to 20g of conjugated linoleic acid or derivative thereof per day.

8. Use as claimed in Claim 1, wherein the conjugated linoleic acid or derivative thereof comprises trans 10, cis 12 and cis9, trans 11 isomers and the weight ratio of trans 10, cis 12 isomer to cis9, trans 11 isomer is at least 1.2:1.

9. Use as claimed in Claim 1 or Claim 8, wherein the conjugated linoleic acid or derivative thereof comprises at least 50% by weight based on the total amount of conjugated linoleic acid of the trans 10, cis 12 isomer.

10. Use as claimed in any one of Claims 1 to 9, wherein the composition is a foodstuff selected from the group consisting of margarines, fat continuous or water continuous or bicontinuous spreads, fat reduced spreads, confectionery products such as chocolate or chocolate coatings or chocolate fillings or bakery fillings, ice creams, ice cream coatings, ice cream inclusions, dressings, mayonnaises, cheeses, cream alternatives, dry soups, drinks, cereal bars, sauces, snack bars, dairy products, clinical nutrition products and infant formulations.

11. Use as claimed in any one of Claims 1 to 9, wherein the composition is a pharmaceutical composition in the form of a tablet, capsule, solution or emulsion.

12. Use as claimed in any one of Claims 1 to 9, wherein the composition is a food supplement in the form of a soft gel or a hard capsule comprising an encapsulating material selected from the group consisting of gelatin, starch, modified starch, starch derivatives such as glucose, sucrose, lactose and fructose.

13. The use of a conjugated fatty acid or a derivative thereof in the manufacture of a composition for the prevention or treatment of the common cold, or the treatment or amelioration of symptoms of the common cold in a mammal, wherein the conjugated fatty acid or derivative thereof is conjugated linoleic acid or a salt, a mono-, di-, tri-glyceride or a C₁-C₆ alkyl ester thereof, the conjugated linoleic acid or derivative thereof comprises trans10, cis12 and cis9, trans11 isomers and the weight ratio of trans 10, cis 12 isomer to cis9, trans 11 isomer is at least 1.2:1.

14. Use as claimed in Claim 13, wherein the conjugated linoleic acid or derivative thereof comprises at least 50% by weight of the trans10, cis12 isomer of conjugated linoleic acid.

## Patentansprüche

1. Verwendung einer konjugierten Linolsäure oder eines Derivats derselben, das aus Salzen, Mono-, Di-, Triglyzeriden und C₁- bis C₆-Alkylestern derselben ausgewählt ist, bei der Herstellung einer Zusammensetzung für die Behandlung oder Verbesserung von Symptomen der gewöhnlichen Erkältung bei einem Säugetier, wobei die konjugierte Linolsäure oder das Derivat derselben eine Mischung von trans10, cis12 und cis9, trans11-Isomeren aufweist.

2. Verwendung wie in Anspruch 1 beansprucht, wobei die Symptome ausgewählt sind aus einem oder mehreren von Niesen, laufender Nase, Nasenverschluss, Halsschmerzen, kratzigem Hals, Husten, Heiserkeit, Asthmaverschlimmerung, Kopfschmerzen, Fieber, Kälte, Unpässlichkeit und Halsschmerzen.

3. Verwendung wie in Anspruch 2 beansprucht, wobei das Symptom Halsschmerzen ist.

4. Verwendung wie in einem der Ansprüche 1 bis 3 beansprucht, wobei das Säugetier ein Mensch ist.

5. Verwendung wie in einem der Ansprüche 1 bis 4 beansprucht, wobei die Zusammensetzung eine pharmazeutische Zusammensetzung, ein Nahrungsmittel oder ein Nahrungsergänzungsmittel ist.

6. Verwendung wie in einem der Ansprüche 1 bis 5 beansprucht, wobei die gewöhnliche Erkältung durch ein Coronavirus oder ein Rhinovirus verursacht ist.

7. Verwendung wie in einem der Ansprüche 1 bis 6 beansprucht, wobei die Zusammensetzung eine Menge an konjugierter Linolsäure oder eines Derivats derselben aufweist, so dass das Säugetier eine Dosis von 0,1 bis 20 g konjugierter Linolsäure oder Derivat derselben pro Tag erhält.

8. Verwendung wie in Anspruch 1 beansprucht, wobei die konjugierte Linolsäure oder das Derivat derselben trans10, cis12- und cis9, trans11-lsomere aufweist und das Gewichtsverhältnis von trans10, cis12-Isomer zu cis9, trans11-Isomer mindestens 1,2:1 beträgt.

9. Verwendung wie in Anspruch 1 oder 8 beansprucht, wobei die konjugierte Linolsäure oder das Derivat derselben basierend auf der Gesamtmenge an konjugierter Linolsäure mindestens 50 Gewichts-% des trans 10, cis 12-Isomers aufweist.

10. Verwendung wie in einem der Ansprüche 1 bis 9 beansprucht, wobei die Zusammensetzung ein Nahrungsmittel ist, das aus der Gruppe ausgewählt ist, welche besteht aus Margarinen, fettkontinuierlichen oder wasserkontinuierlichen oder bikontinuierlichen Aufstrichen, fettreduzierten Aufstrichen, Konfektprodukten, wie beispielsweise Schokolade oder Schokoladebeschichtungen oder Schokoladefüllungen oder Backfüllungen, Eiscremes, Eiscremebeschichtungen, Einscremeeinschlüssen, Dressings, Majonäsen, Käsen, Sahnealternativen, Trockensuppen, Getränken, Getreideriegeln, Saucen, Snackriegeln, Molkereiprodukten, klinischen Nahrungsprodukten und Kinderformulierungen.

11. Verwendung wie in einem der Ansprüche 1 bis 9 beansprucht, wobei die Zusammensetzung eine pharmazeutische Zusammensetzung in Form einer Tablette, Kapsel, Lösung oder Emulsion ist.

12. Verwendung nach einem der Ansprüche 1 bis 9, wobei die Zusammensetzung ein Nahrungsergänzungsmittel in der Form eines weichen Gels oder einer harten Kapsel ist, die ein einkapselndes Material aufweist, welches aus der Gruppe ausgewählt ist, die besteht aus Gelatine, Stärke, modifizierter Stärke, Stärkederivaten, wie beispielsweise Glukose, Sukrose, Laktose und Fruktose.

13. Verwendung einer konjugierten Fettsäure oder eines Derivats derselben bei der Herstellung einer Zusammensetzung für die Verhinderung oder Behandlung der gewöhnlichen Erkältung oder der Behandlung oder Verbesserung von Symptomen der gewöhnlichen Erkältung bei einem Säugetier, wobei die konjugierte Fettsäure oder das Derivat derselben eine konjugierte Linolsäure oder ein Salz, ein Mono-, Di-, Triglyzerid oder ein C₁- bis C₆-Alkylester derselben ist, wobei die konjugierte Linolsäure oder das Derivat derselben trans10, cis12- und cis9, trans11-Isomere aufweist und das Gewichtsverhältnis von trans10, cis12-Isomer zu cis9, trans11-Isomer mindestens 1,2:1 beträgt.

14. Verwendung wie in Anspruch 13 beansprucht, wobei die konjugierte Linolsäure oder das Derivat derselben mindestens 50 Gewichts-% des trans10, cis12-Isomers der konjugierten Linolsäure aufweist.

## Revendications

1. Utilisation d'acide linoléique conjugué ou d'un dérivé de celui-ci choisi parmi les sels, les mono-, di-, tri-glycérides et les esters d'alkyle en C₁ à C₆ de celui-ci dans la fabrication d'une composition pour le traitement ou l'amélioration de symptômes du rhume de cerveau chez un mammifère, dans laquelle l'acide linoléique conjugué ou le dérivé de celui-ci comprend un mélange d'isomères trans10, cis12 et cis9, trans11.

2. Utilisation selon la revendication 1, dans laquelle les symptômes sont choisis parmi un ou plusieurs parmi un éternuement, une rhinorrhée, une obstruction nasale, un mal de gorge, un prurit pharyngé, une toux, un enrouement, une crise d'asthme, un mal de tête, une fièvre, des frissons, un malaise et un mal de gorge.

3. Utilisation selon la revendication 2, dans laquelle le symptôme est un mal de gorge.

4. Utilisation selon l'une quelconque des revendications 1 à 3, dans laquelle le mammifère est un être humain.

5. Utilisation selon l'une quelconque des revendications 1 à 4, dans laquelle la composition est une composition pharmaceutique, une denrée alimentaire ou un complément alimentaire.

6. Utilisation selon l'une quelconque des revendications 1 à 5, dans laquelle le rhume de cerveau est provoqué par un coronavirus ou un rhinovirus.

7. Utilisation selon l'une quelconque des revendications 1 à 6, dans laquelle la composition comprend une quantité d'acide linoléique conjugué ou d'un dérivé de celui-ci telle que le mammifère reçoit une dose de 0,1 à 20 g d'acide linoléique conjugué ou de dérivé de celui-ci par jour.

8. Utilisation selon la revendication 1, dans laquelle l'acide linoléique conjugué ou le dérivé de celui-ci comprend les isomères trans10, cis12 et cis9, trans11 et le rapport pondéral de l'isomère trans10, cis12 à l'isomère cis9, trans11 est d'au moins 1,2:1.

9. Utilisation selon la revendication 1 ou la revendication 8, dans laquelle l'acide linoléique conjugué ou le dérivé de celui-ci comprend au moins 50 % en poids, sur la base de la quantité totale d'acide linoléique conjugué, de l'isomère trans10, cis12.

10. Utilisation selon l'une quelconque des revendications 1 à 9, dans laquelle la composition est une denrée alimentaire choisie dans le groupe constitué par les margarines, les produits à tartiner gras continus ou aqueux continus ou bicontinus, les produits à tartiner maigres, les produits de confiserie tels que le chocolat ou les enrobages de chocolat ou les garnissages de chocolat ou les garnissages de boulangerie, les crèmes glacées, les enrobages pour crème glacée, les inclusions pour crème glacée, les vinaigrettes, les mayonnaises, les fromages, les substituts de crème, les soupes en poudre, les boissons, les barres céréalières, les sauces, les casse-croûte, les produits laitiers, les produits de nutrition clinique et les préparations pour nourrissons.

11. Utilisation selon l'une quelconque des revendications 1 à 9, dans laquelle la composition est une composition pharmaceutique sous la forme d'un comprimé, d'une capsule, d'une solution ou d'une émulsion.

12. Utilisation selon l'une quelconque des revendications 1 à 9, dans laquelle la composition est un complément alimentaire sous la forme d'un gel mou ou d'une capsule dure comprenant un matériau d'encapsulation choisi dans le groupe constitué par la gélatine, l'amidon, un amidon modifié, les dérivés d'amidon tels que le glucose, le saccharose, le lactose et le fructose.

13. Utilisation d'un acide gras conjugué ou d'un dérivé de celui-ci dans la fabrication d'une composition pour la prévention ou le traitement du rhume de cerveau, ou pour le traitement ou l'amélioration de symptômes du rhume de cerveau chez un mammifère, dans laquelle l'acide gras conjugué ou le dérivé de celui-ci est l'acide linoléique conjugué ou un sel, un mono-, di-, tri-glycéride ou un ester d'alkyle en C₁ à C₆ de celui-ci, l'acide linoléique conjugué ou le dérivé de celui-ci comprend les isomères trans10, cis12 et cis9, trans11 et le rapport pondéral de l'isomère trans10, cis12 à l'isomère cis9, trans11 est d'au moins 1,2:1.

14. Utilisation selon la revendication 13, dans laquelle l'acide linoléique conjugué ou le dérivé de celui-ci comprend au moins 50 % en poids de l'isomère trans10, cis12 de l'acide linoléique conjugué.
